(19) Europäisches Patentamt
European Patent Office
Office européen des brevets

(11) Veröffentlichungsnummer : **0 348 718 B1**

(12) **EUROPÄISCHE PATENTSCHRIFT**

(45) Veröffentlichungstag der Patentschrift :
**17.03.93 Patentblatt 93/11**

(51) Int. Cl.[5] : **A61K 6/00,** A61K 31/66, A61K 7/16

(21) Anmeldenummer : **89110532.2**

(22) Anmeldetag : **10.06.89**

# (54) Flüssigkeit zur Konditionierung von Zahn- oder Knochensubstanz.

(30) Priorität : **25.06.88 DE 3821578**

(43) Veröffentlichungstag der Anmeldung :
**03.01.90 Patentblatt 90/01**

(45) Bekanntmachung des Hinweises auf die Patenterteilung :
**17.03.93 Patentblatt 93/11**

(84) Benannte Vertragsstaaten :
**AT BE CH DE ES FR GB GR IT LI LU NL SE**

(56) Entgegenhaltungen :
**EP-A- 0 161 386**
**DE-A- 2 360 797**
**FR-A- 2 361 865**

(73) Patentinhaber : **BAYER AG**
**W-5090 Leverkusen 1 Bayerwerk (DE)**

(72) Erfinder : **Podszun, Wolfgang, Dr.**
**Roggendorfstr. 55**
**W-5000 Köln 80 (DE)**
Erfinder : **Müller, Michael, Dr.**
**Richard Zanders-Str. 34**
**W-5060 Bergisch Gladbach 2 (DE)**
Erfinder : **Winkel, Jens, Dr.**
**Letterhaus Strasse 1**
**W-5000 Köln-Pesch (DE)**
Erfinder : **Block, Hans-Dieter, Dr.**
**Biesenbach 49**
**W-5090 Leverkusen 3 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft eine Flüssigkeit zur Konditionierung von defekter Zahn- oder Knochensubstanz für eine Versorgung mit plastischem Kunststoffmaterial.

Besonders im Dentalbereich werden härtende plastische Kunststoffmaterialien als Füllungsmaterialien bei der Versorgung von Kavitäten an der Zahn- oder Knochensubstanz verwendet. Als härtende Kunststoffmaterialien werden im allgemeinen Füllungen auf Acrylatbasis bevorzugt. Diese polymeren Füllungen haben jedoch den Nachteil, daß sie schlecht auf dem Zahnbein oder dem Knochen haften bleiben. Um dieses Problem zu lösen, hat man z.B. bisher teilweise Unterschneidungen vorgenommen; dazu war es erforderlich, über den angegriffenen Bereich hinaus, beachtliche Mengen an frischem Zahnbein zu entfernen.

Um diese Nachteile zu vermeiden, hat man das Zahnbein oder den Knochen auf verschiedene Weise vorbehandelt, um die Haftung des Kunststoffmaterials zu erhöhen.

So ist es bekannt, das Zahnbein oder die Schmelzoberfläche mit starken Säuren, z.B. Phosphorsäure, anzuätzen und dann die Füllung vorzunehmen (Scand. J. Dental Res. 88, 348-351 (1981)). Abgesehen von der Reizwirkung der starken Säure im Mundbereich, ist die Haftung der Füllung unzureichend.

Es ist außerdem bekannt, das Zahnbein mit Ethylendiamintetraessigsäure (EDTA) vorzubehandeln und dann mit einem Beschichtungsmittel aus einem aliphatischen Aldehyd oder einem Keton und einem olefinisch ungesättigten Monomer, z.B. einem Ester der Acryl- oder Methacrylsäure, zu versehen (EP-A-0 141 324 und EP-A-0 109 057).

Es wurden Flüssigkeiten zur Konditionierung von Zahn- oder Knochensubstanz gefunden, die in wäßriger Lösung Phosphonocarbonsäuren der Formel

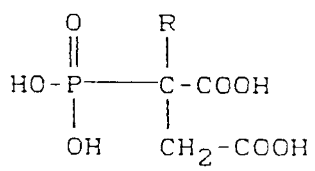

(I),

in der

R Wasserstoff oder gegebenenfalls durch Hydroxy, Carboxy und/oder die Gruppen

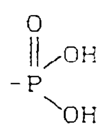

und oder -COOR′

substituiertes $C_1$-bis $C_{12}$-Alkyl, $C_2$-bis $C_{12}$-Alkenyl, $C_5$-bis $C_8$-Cycloalkyl, $C_6$-bis $C_{12}$-Aryl oder $C_7$-bis $C_{12}$-Aralkyl

wobei

R′ für Wasserstoff oder für einen $C_1$ bis $C_{12}$-Alkylrest steht, bedeutet,

und/oder deren Salze,

wobei die Lösung einen pH-Wert im Bereich von 1 bis 8 aufweist,

enthalten.

Die neuen erfindungsgemäßen Flüssigkeiten konditionieren die Zahn- oder Knochensubstanz vor einer Beschichtung mit einem Grundierungsmittel (Primer oder Liner). Auf der auf diese Weise vorbehandelten Zahn- oder Knochensubstanz tritt eine feste Bindung eines aufgetragenen plastischen Kunststoffmaterials ein.

Im Rahmen der vorliegenden Erfindung haben die Reste im allgemeinen die folgende Bedeutung:

Alkyl steht im allgemeinen für einen geradkettigen oder verzweigten Kohlenwasserstoffrest mit 1 bis 12 Kohlenstoffatomen. Bevorzugt wird Niederalkyl mit 1 bis etwa 6 Kohlenstoffatomen, Beispielsweise seien Methyl, Ethyl, Propyl, Isopropyl, Butyl, Isobutyl, Pentyl, Isopentyl, Hexyl und Isohexyl genannt. Insbesondere bevorzugt werden Methyl und Ethyl.

Alkenyl steht im allgemeinen für einen geradkettigen oder verzweigten Kohlenwasserstoffrest mit 2 bis 12 Kohlenstoffatomen und einer oder zwei, bevorzugt einer, Doppelbindung. Bevorzugt wird Niederalkenyl mit 2 bis etwa 6 Kohlenstoffatomen. Beispielsweise seien Allyl, Vinyl, Propenyl, Isopropenyl, Butenyl, Isobutenyl, Pentenyl, Isopentenyl, Hexenyl und Isohexenyl genannt.

Cycloalkyl steht im allgemeinen für einen cyclischen Kohlenwasserstoffrest mit 5 bis 8 Kohlenstoffatomen. Beispielsweise seien Cyclopentyl, Cyclohexyl, Cycloheptyl und Cyclooctyl genannt. Insbesondere bevorzugt

werden Cyclopentyl und Cyclohexyl.

Aryl steht im allgemeinen für einen aromatischen Rest mit 6 bis etwa 12 Kohlenstoffatomen. Bevorzugte Arylreste sind Phenyl, Naphthyl und Biphenylyl.

Aralkyl steht im allgemeinen für einen über eine Alkylkette gebundenen Arylrest mit 7 bis 12 Kohlenstoffatomen. Bevorzugt werden Aralkylreste mit 1 bis 5 Kohlenstoffatomen im aliphatischen und 6 bis 12 Kohlenstoffatomen im aromatischen Teil. Beispielsweise seien die folgenden Aralkylreste genannt: Benzyl, Naphthylmethyl, Phenethyl und Phenylpropyl.

Im Rahmen der vorliegenden Erfindung werden Phosphonocarbonsäuren der Formel (I) bevorzugt, worin

R Wasserstoff oder gegebenfalls durch -COOH oder

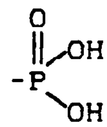

substituiertes $C_1$ bis $C_6$-Alkyl

bedeutet.

Beispielsweise seien die folgenden Phosphonocarbonsäuren genannt:

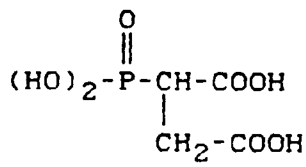

Phosphonobernsteinsäure (PBS)

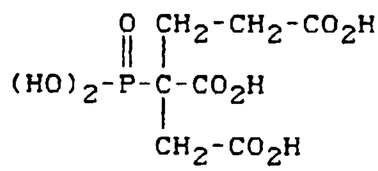

2-Phosphonobutan-1,2,4-tricarbonsäure (PBTS)

und

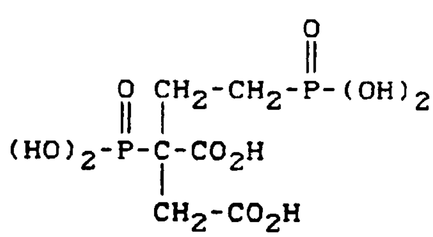

2,4-Diphosphonobutan-1,2-dicarbonsäure (DPBD).

Als Salze der erfindungsgemäßen Phosphonocarbonsäuren seien insbesondere die Alkalisalze, bevorzugt die Natrium- und Kaliumsalze, genannt.

Der Anteil der Phosphonocarbonsäure bzw. deren Salze in den erfindungsgemäßen Flüssigkeiten zur Konditionierung von Zahn- oder Knochensubstanzen wird durch den pH-Wert festgelegt.

Erfindungsgemäß liegt der pH-Wert im Bereich von 1 bis 8, bevorzugt im Bereich von 2 bis 7, insbesondere bevorzugt im Bereich von 2,5 bis 5.

Die Einstellung des pH-Wertes ist durch Zusatz von Laugen, beispielsweise von Natriumhydroxid oder Kaliumhydroxid möglich. Die Messung des pH-Wertes kann in an sich bekannter Weise, z.B. mit Hilfe geeigneter Indikatoren oder mit Hilfe von potentiometrischen Meßverfahren (Ullman, Band 5, 926 bis 936 (1980)) erfolgen.

Die erfindungsgemäßen wäßrigen Flüssigkeiten zur Konditionierung von Zahn- oder Knochensubstanzen können als weiteren Bestandteil organische Carbonsäuren mit einem pKs-Wert von kleiner als 5, bevorzugt im Bereich von 1 bis 4, enthalten. Beispielsweise seien die folgenden Carbonsäuren genannt: Brenztraubensäure, Zitronensäure und Oxalsäure.

Weiterhin können die erfindungsgemäßen Flüssigkeiten amphotere Aminoverbindungen mit einem pKs-Wert von 9,0 bis 10,6 und einem $pK_B$-Wert von 11,5 bis 12,5 enthalten.

Vorzugsweise seien amphotere Aminoverbindungen der Formel (II)

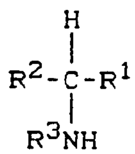

(II),

in der

$R^1$ für eine Carboxylgruppe steht,

$R^2$ Wasserstoff, gegebenenfalls durch Hydroxy, Mercapto, Methylthio, Carboxy, Carbonamid, Amino, Phenyl, Hydroxy-phenyl oder die Gruppen

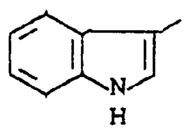 , 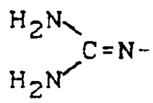 , 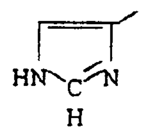

substituierter Niederalkylrest und

$R^3$ Wasserstoff oder Phenyl bedeutet,

$R^1$ und $R^3$ durch einen Propylenrest verbunden sind

oder in der

$R^1$ für Wasserstoff steht,

$R^2$ die Gruppe

$$-A-NH_3X$$

in der

A für einen Alkylenrest mit 1 bis 6 Kohlenstoffatomen und

X für Halogen steht,

bedeutet und

$R^3$ Wasserstoff bedeutet,

genannt.

Beispielsweise seien die folgenden amphoteren Aminoverbindungen genannt: Glycin, Serin, Threonin, Cystein, Tyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrobromid, Propylendiaminhydrochlorid, Propylendiaminhydrobromid, Butylendiaminhydrochlorid und Butylendiaminhydrobromid.

Besonders bevorzugte amphotere Aminoverbindungen sind Glycin, Phenylalanin, Lysin und Ethylendiaminhydrochlorid.

Auch der Zusatz von Komplexbildnern wie Ethylendiamintetraessigsäure (EDTA), und davon abgeleitete Salze ist möglich.

Die erfindungsgemäßen wäßrigen Flüssigkeiten zur Konditionierung können beispielsweise auf 100 Gew.-Teile

| | |
|---|---|
| 1 bis 50 | Gew.-Teile Phosphonocarbonsäuren, oder davon abgeleitete Salze |
| 0 bis 15 | Gew.-Teile an organischen Carbonsäuren, |
| 0 bis 10 | Gew.-Teile an amphoteren Aminoverbindungen, |
| 0 bis 10 | Gew.-Teile an Ethylendiamintetraessigsäure, oder davon abgeleitete Salze enthalten. |

Es wurde auch ein Verfahren zur Herstellung von Flüssigkeiten zur Konditionierung von Zahn- oder Knochensubstanzen gefunden, das dadurch gekennzeichnet ist, daß eine wäßrige Lösung einer Phosphonocarbonsäure der Formel

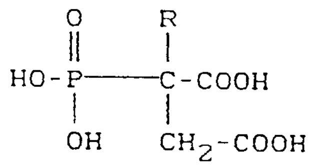

(I),

in der

R Wasserstoff oder gegebenenfalls durch Hydroxy, Carboxy und/oder die Gruppen

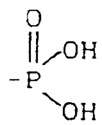

und/oder -COOR′

substituiertes $C_1$-bis $C_{12}$-Alkyl, $C_2$-bis $C_{12}$-Alkenyl, $C_5$- bis $C_8$-Cycloalkyl, $C_6$-bis $C_{12}$-Aryl oder $C_7$ bis $C_{12}$-Aralkyl

wobei

R′ für Wasserstoff oder für einen $C_1$ bis $C_{12}$-Alkylrest steht, bedeutet,

und/oder deren Salze,

hergestellt wird, wobei ein pH-Wert im Bereich von 1 bis 8 eingehalten wird.

Die Einstellung des pH-Wertes erfolgt im allgemeinen durch Zusätze von Laugen, beispielsweise von Natriumhydroxid oder Kaliumhydroxid.

Die Komponenten werden im allgemeinen bei der Herstellung der erfindungsgemäßen Flüssigkeiten unter kräftigem Rühren zusammengegeben, Die Zusammengabe der Komponenten erfolgt im allgemeinen bei Raumtemperatur, beispielsweise im Temperaturbereich von 0 bis 30°C.

Die erfindungsgemäßen Konditionierflüssigkeiten können darüber hinaus Verdickungsmittel oder oberflächenaktive Substanzen enthalten, um ihre Viskosität oder ihr Benetzungsverhalten speziellen Anforderungen anzupassen. Besonders günstig ist ein Zusatz von Polyvinylpyrrolidon oder Polyethylenglykolen.

Bei der Anwendung trägt man die erfindungsgemäßen Flüssigkeiten auf die defekte Zahn- oder Knochensubstanz, beispielsweise in eine Kavität, auf. Bevorzugt seien hier Kavitäten im Zahnschmelz (Enamel) oder Zahnbein (Dentin) genannt.

Nach dem Auftragen der erfindungsgemäßen Flüssigkeiten werden sie im allgemeinen, z.B. mit warmer Luft, getrocknet.

Vor der Versorgung der defekten Zahn- oder Knochensubstanz mit dem plastischen Kunststoffmaterial erfolgt nach der Konditionierung mit den erfindungsgemäßen Flüssigkeiten vorzugsweise eine Beschichtung mit einem Grundierungsmaterial.

Es seien hier besonders Grundierungsmaterialien genannt wie sie in der EP-A-0 141 324 und der EP-A-0 199 057 beschrieben werden.

Besonders bevorzugt werden Grundierungsmaterialien, die einen Aldehyd oder ein Keton und ein ungesättigtes Monomer mit aktivem Wasserstoff enthalten. Diese Materialen sind von dem Schutzbereich der vorliegenden Erfindung ausgeschlossen.

Als Aldehyde seien hier Formaldehyd, Verbindungen die Formaldehyd freisetzen können, Acetaldehyd, Propionaldehyd, Butyraldehyd und Glutaraldehyd genannt. Insbesondere bevorzugt ist der Glutaraldehyd.

Als Ketone seien hier Cyclopentanon, Benzophenon, Cyclohexanon, 2,4-Pentandion und Kamperchinon genannt. Insbesondere bevorzugt wird Kampferchinon.

Als olefinisch ungesättigte Monomere mit aktivem Wasserstoff (Bronsted-Säure) seien Acrylsäureester, Methacrylsäureester und Acrylsäure- bzw. Methacrylsäureurethane mit OH-, $NH_2$-, NH-, SH- oder PH-Gruppen genannt. Insbesondere bevorzugt wird das Hydroxyethylmethacrylat.

Insbesondere bevorzugt sind Grundierungsmaterialien, die 1 bis 50 Gew.-% eines aliphatischen Aldehyds mit 1 bis 20 Kohlenstoffatomen und 5 bis 80 Gew.-% eines olefinisch ungesättigten Monomers mit wenigstens einem aktiven Wasserstoffatom in Form von OH-, $NH_2$- oder SH-Gruppen und gegebenenfalls Wasser und/oder ein toxikologisch akzeptables organisches Lösungsmittel enthalten. Die härtenden Kunststoffmaterialien werden im wesentlichen durch das Anwendungsgebiet bestimmt. So können beispielsweise im Dentalbereich für die Polymerisation nur Monomere eingesetzt werden, die physiologisch unbedenklich sind und die im Mundbereich polymerisieren können. Solche Monomeren für Zahnfüllungen sind an sich bekannt (z.B. Ullmanns Enzyklopädie der Technischen Chemie).

Als Kunststoffmaterialien seien beispielsweise Massen aus Acrylat- und/oder Methacrylatmonomeren, geeigneten Katalysatoren, Startern, Beschleunigern und Füllstoffen genannt.

Durch die Konditionierung der defekten Zahn- oder Knochensubstanz mit den erfindungsgemäßen Flüssigkeiten erhält man überraschenderweise eine Basis für die Versorgung mit Kunststoffmaterialien, die eine hohe Haltbarkeit und Festigkeit der Reparatur gewährleistet.

Beispiel 1

Konditionierflüssigkeiten

Aus Phosphonobernsteinsäure (PBS), Glycin und Wasser wurden Lösungen der unten angegebenen Konzentrationen und pH-Werte hergestellt. Die Einstellung des pH-Wertes erfolgte bei den Beispielen B-D mit 4N-NaOH.

| Beispiel Nr. | PBS [Mol/l] | Glycin [Mol/l] | pH-Wert |
|---|---|---|---|
| 1 A | 2,40 | 0,057 | <1 |
| 1 B | 0,44 | 0,057 | 3,5 |
| 1 C | 0,44 | 0,057 | 6,0 |
| 1 D | 0,44 | 0,057 | 8,0 |
| 1 E | 2,40 | 0,316 | 3,5 |

Beispiel 2

Konditionierflüssigkeiten

Aus 2-Phosphonobutan-1,2,4-tricarbonsäure (PBTS), Glycin und Wasser wurden Lösungen der unten angegebenen Konzentrationen und pH-Werte hergestellt. Die Einstellung des pH-Wertes erfolgte bei den Beispielen B-D mit 4N-NaOH.

| Beispiel Nr. | PBTS [Mol/l] | Glycin [Mol/l] | pH-Wert |
|---|---|---|---|
| 2 A | 2,3 | 0,057 | <1 |
| 2 B | 0,46 | 0,057 | 3,5 |
| 2 C | 0,46 | 0,057 | 6,0 |
| 2 D | 0,46 | 0,057 | 8,0 |
| 2 E | 2,1 | 0,323 | 3,5 |

Beispiel 3

(Prüfung der Bindungsstärke)

In dem Beispiel wird die Bindungsstärke (Zugfestigkeit) zwischen Zahnbein (Dentin) bzw. Zahnschmelz (Enamel) und einer handelsüblichen Kunststoffüllmasse gemessen.

Für den Test werden herausgezogene und im feuchten Zustand aufbewahrte Menschenzähne benutzt. Die Zähne werden durch Guß in Epoxidharz eingelagert; durch Naßschleifen wird eine flache Oberfläche erzeugt. Das abschließende Schleifen erfolgt mit Carborundumpapier 1000.

Daraufhin wird die Oberfläche mit einer Flüssigkeit aus Beispiel 1 oder 2 jeweils 60 Sekunden lang behandelt. Danach wird die behandelte Stelle mit destilliertem Wasser gespült und mit Luft getrocknet.

Anschließend wird die mit den Flüssigkeiten der Beispiele 1 oder 2 konditionierte Fläche 60 Sekunden lang mit dem Beschichtungsmittel aus 5 Gew.-% Glutardialdehyd, 36 Gew.-% Hydroxyethylmethacrylat und 59 Gew.-% Wasser behandelt. Danach wird die Fläche mit Luft getrocknet.

Zur Herstellung eines Probekörpers zum Messen der Bindungsstärke wird eine zylindrische, gespaltete Teflonform auf die vorstehend beschriebene, behandelte Oberfläche gespannt (Scand. J. Dent. Res. 88, 348 bis 351 (1981)).

In die form wird ein dünner Film aus einer Lösung aus 65 Gew.-% Bisphenol-A-diglycidylmethacrylat, 34,3 Gew.-% Triethylenglykoldimethacrylat, 0,2 Gew.-% Kampferchinon, 0,5 Gew.-% Sulfonamid aufgebracht und anschließend ein lichtaktiviertes Kunststoffüllungsmaterial (Lumifor, Bayer AG) eingefüllt. Es wird 60 Sekunden mit einer Fotopolymerisationslampe ausgehärtet. Nach weiteren 15 Minuten wird die Teflonform abgenommen und die Bestimmung der Bindungsstärke mit Hilfe einer Instron-Zugversuchsapparatur (Scand. J. Dent. Res. 88, 348 bis 351 (1981)) bei einer Abzugsgeschwindigkeit von 5 mm/Min. durchgeführt.

Folgende Zugfestigkeiten an Zahnbein wurden dabei ermittelt:

| Konditionierflüssigkeit | Zugfestigkeit [N/mm²] |
|---|---|
| 1 A | 6,1 ± 0,4 |
| 1 B | 17,3 ± 2,9 |
| 1 C | 20,4 ± 2,3 |
| 1 D | 13,9 ± 3,1 |
| 1 E | 17,5 ± 1,2 |
| 2 A | 6,0 ± 1,7 |
| 2 B | 17,6 ± 1,7 |
| 2 C | 16,7 ± 0,6 |
| 2 D | 17,8 ± 3,1 |
| 2 E | 18,1 ± 4,0 |

Beispiel 4

Aus 2,4-Diphosphonobutan-1,2-dicarbonsäure (DPBD), Glycin, 4N-NaOH und Wasser wurde eine Lösung mit folgender Zusammensetzung hergestellt:

| DPBD [Mol/l] | Glycin [Mol/l] | pH-Wert |
|---|---|---|
| 0,45 | 0,057 | 3,5 |

Nach der im Beispiel 3 beschriebenen Methode wurde die Bindungsfestigkeit auf Schmelz und Zahnbein ermittelt. Es wurden die folgenden Werte erhalten:

Zahnschmelz: 14,3 N/mm² Zahnbein: 13,5 N/mm²

Beispiel 5

Aus 2-Phosphonobutan-1,2,4-tricarbonsäure (PBTS), Citronensäure (CS), EDTA-Magnesiumsalz (Mg-EDTA) und Polyethylenglykol-4000 (PEG) wurden wäßrige Konditionierflüssigkeiten hergestellt und Bindungsfestigkeiten nach Beispiel 3 bestimmt.

| Beispiel Nr. | 5 A | 5 B |
|---|---|---|
| PBTS [Gew.-%] | 1 | 5 |
| MgEDTA [Gew.-%] | 1 | 1 |
| CS [Gew.-%] | 5 | 5 |
| PEG [Gew.-%] | 10 | 10 |
| Bindungsfestigkeit auf Zahnbein [N/mm²] | 17,7±1,4 | 19,8±2,7 |
| Bindungsfestigkeit auf Zahnschmelz [N/mm²] | 15,1±4,7 | 16,0±3,7 |

## Patentansprüche

### Patentansprüche für folgende Vertragsstaaten : AT, BE, CH, DE, FR, GB, GR, IT, LI, LU, NL, SE

1. Flüssigkeiten zur Kontidionierung von Zahn- oder Knochensubstanzen, enthaltend in wäßriger Lösung Phosphonocarbonsäuren der Formel

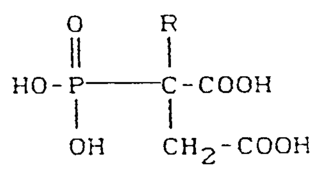

(I),

in der

R Wasserstoff oder gegebenenfalls durch Hydroxy, Carboxy und/oder die Gruppen

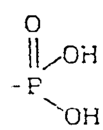

und/oder -COO'R

substituiertes $C_1$-bis $C_{12}$-Alkyl, $C_2$-bis $C_{12}$-Alkenyl, $C_5$- bis $C_8$-Cycloalkyl, $C_6$-bis $C_{12}$-Aryl oder $C_7$-bis $C_{12}$-Aralkyl

wobei

R' für Wasserstoff oder für einen $C_1$ bis $C_{12}$-Alkylrest steht, bedeutet,

und/oder deren Salze,

wobei die Lösung einen pH-Wert im Bereich von 1 bis 8 aufweist.

2. Flüssigkeiten nach Anspruch 1, dadurch gekennzeichnet, daß sie einen pH-Wert im Bereich von 2 bis 7 aufweisen.

3. Flüssigkeiten nach Ansprüchen 1 und 2, dadurch gekennzeichnet, daß sie eine organische Carbonsäure

mit einem pKs-Wert von kleiner als 5 enthalten,

4. Flüssigkeiten nach den Ansprüchen 1 bis 3, dadurch gekennzeichnet, daß sie eine amphotere Aminoverbindung mit einem pKs-Wert im Bereich von 9,0 bis 10,6 und mit einem $pK_B$-Wert im Bereich von 11,5 bis 12,5 enthalten.

5. Flüssigkeiten nach den Ansprüchen 1 bis 4, dadurch gekennzeichnet, daß sie Ethylendiamintetraessigsäure und/oder deren Salze enthalten.

6. Verfahren zur Herstellung von Flüssigkeiten zur Konditionierung von Zahn- oder Knochensubstanz, dadurch gekennzeichnet, daß eine wäßrige Lösung von Phosphonocarbonsäuren der Formel

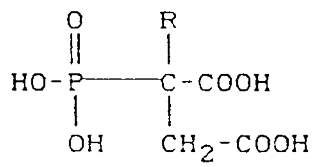

(I),

in der
R Wasserstoff oder gegebenenfalls durch Hydroxy, Carboxy und/oder die Gruppen

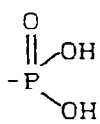

und/oder -COOR′
substituiertes $C_1$-bis $C_{12}$-Alkyl, $C_2$-bis $C_{12}$-Alkenyl, $C_5$-bis $C_8$-Cycloalkyl, $C_6$-bis $C_{12}$-Aryl oder $C_7$-bis $C_{12}$-Aralkyl
wobei
R′ für Wasserstoff oder für einen $C_1$ bis $C_{12}$-Alkylrest steht, bedeutet,
und/oder deren Salze,
hergestellt wird, wobei ein pH-wert im Bereich von 1 bis 8 eingehalten wird.

7. Verwendung von Flüssigkeiten nach den Ansprüchen 1 bis 5 als Basis zur Herstellung von Mitteln für eine Versorgung von Zahn- oder Knochensubstanzkavitäten mit Kunststoffmaterial.

**Patentansprüche für folgenden Vertragsstaat : ES**

1. Verfahren zur Herstellung von Flüssigkeiten zur Konditionierung von Zahn- oder Knochensubstanz, dadurch gekennzeichnet, daß eine wäßrige Lösung von Phosphonocarbonsäuren der Formel

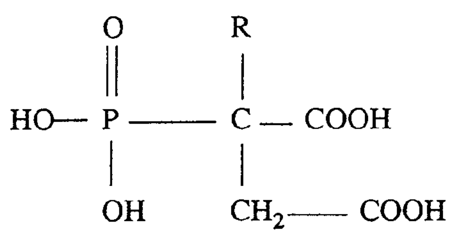

(I),

in der
R Wasserstoff oder gegebenenfalls durch Hydroxy, Carboxy und/oder die Gruppen

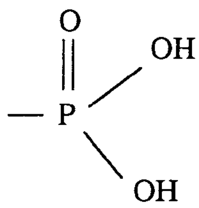

und/oder -COOR′

substituiertes $C_1$-bis $C_{12}$-Alkyl, $C_2$-bis $C_{12}$Alkenyl, $C_5$-bis $C_8$-Cycloalkyl, $C_6$-bis $C_{12}$-Aryl oder $C_7$-bis $C_{12}$-Aralkyl

wobei

R′ für Wasserstoff oder für einen $C_1$ bis $C_{12}$-Alkyrest steht, bedeutet,

und/oder deren Salze,

hergestellt wird, wobei ein pH-Wert im Bereich von 1 bis 8 eingehalten wird.

2. Verfahren nach Anspruch 1, dadurch gekennzeichnet, daß ein pH-Wert im Bereich von 2 bis 7 eingehalten wird.

3. Verfahren nach Ansprüchen 1 und 2, dadurch gekennzeichnet, daß die Flüssigkeit eine organische Carbonsäure mit einem pKs-Wert von kleiner als 5 enthält.

4. Verfahren nach Ansprüchen 1 bis 3, dadurch gekennzeichnet, daß die Flüssigkeit eine amphotere Aminoverbindung mit einem pKs-Wert im Bereich von 9,0 bis 10,6 und mit einem $pK_B$-Wert im Bereich von 11,5 bis 12,5 enthält.

5. Verfahren nach den Ansprüchen 1 bis 4, dadurch gekennzeichnet, daß die Flüssigkeit Ethylendiamintetraessigsäure und/oder deren Salze enthält.

**Claims**

**Claims for the following Contracting States : AT, BE, CH, DE, FR, GB, GR, IT, LI, LU, NL, SE**

1. Liquids for conditioning tooth or bone substances, containing phosphono carboxylic acids of the formula

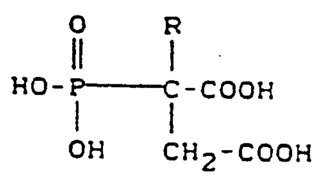

(I),

in which

R denotes hydrogen or, optionally substituted by hydroxyl, carboxyl and/or the groups

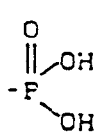

and/or -COOR′,

$C_1$- to $C_{12}$-alkyl, $C_2$- to $C_{12}$-alkenyl, $C_5$- to $C_8$-cycloalkyl, $C_6$- to $C_{12}$-aryl or $C_7$- to $C_{12}$-aralkyl,

where

R′ represents hydrogen or a $C_1$- to $C_{12}$-alkyl radical,

and/or the salts thereof, in aqueous solution, the solution having a pH in the range 1 to 8.

2. Liquids according to Claim 1, characterised in that they have a pH in the range 2 to 7.

3. Liquids according to Claims 1 and 2, characterised in that they contain an organic carboxylic acid having a $pK_a$ below 5.

4. Liquids according to Claims 1 to 3, characterised in that they contain an amphoteric amino compound having a $pK_a$ in the range 9.0 to 10.6 and having a $pK_B$ in the range 11.5 to 12.5.

5. Liquids according to Claims 1 to 4, characterised in that they contain ethylenediaminetetraacetic acid and/or the salts thereof.

6. Process for the preparation of liquids for conditioning tooth or bone substance, characterised in that an aqueous solution of phosphono carboxylic acids of the formula

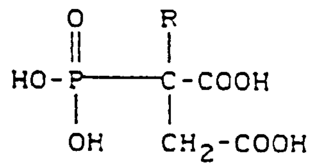

(I),

in which
R denotes hydrogen or, optionally substituted by hydroxyl, carboxyl and/or the groups

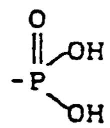

and/or -COOR′,
$C_1$- to $C_{12}$-alkyl, $C_2$- to $C_{12}$-alkenyl, $C_5$-to $C_8$-cycloalkyl, $C_6$- to $C_{12}$-aryl or $C_7$- to $C_{12}$-aralkyl, where
R′ represents hydrogen or a $C_1$- to $C_{12}$-alkyl radical,
and/or the salts thereof, is prepared with the pH being maintained in the range 1 to 8.

7. Use of liquids according to Claims 1 to 5 as basis for preparing agents for management of cavities in tooth or bone substance with synthetic material.

**Claims for the following Contracting State : ES**

1. Process for the preparation of liquids for conditioning tooth or bone substance, characterised in that an aqueous solution of phosphono carboxylic acids of the formula

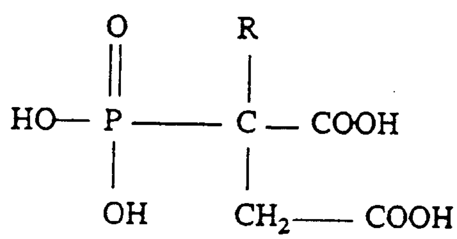

(I)

in which
R denotes hydrogen or, optionally substituted by hydroxyl, carboxyl and/or the groups

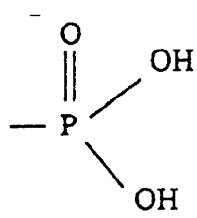

and/or -COOR',

$C_1$- to $C_{12}$-alkyl, $C_2$- to $C_{12}$-alkenyl, $C_5$- to $C_8$-cycloalkyl, $C_6$- to $C_{12}$-aryl or $C_7$- to $C_{12}$-aralkyl, where

R' represents hydrogen or a $C_1$ to $C_{12}$-alkyl radical,

and/or the salts thereof, is prepared with the pH being maintained in the range 1 to 8.

2. Process according to Claim 1, characterised in that a pH in the range 2 to 7 is maintained.

3. Process according to Claims 1 and 2, characterised in that the liquid contains an organic carboxylic acid having a $pK_a$ below 5.

4. Process according to Claims 1 to 3, characterised in that the liquid contains an amphoteric amino compound having a $pK_a$ in the range 9.0 to 10.6 and having a $pK_B$ in the range 11.5 to 12.5.

5. Process according to Claims 1 to 4, characterised in that the liquid contains ethylenediaminetetraacetic acid and/or the salts thereof.

## Revendications

### Revendications pour les Etats contractants suivants : AT, BE, CH, DE, FR, GB, GR, IT, LI, LU, NL, SE

1. Liquides pour la préparation de la matière des dents ou des os à une restauration, contenant en solution aqueuse des acides phosphonocarboxyliques de formule

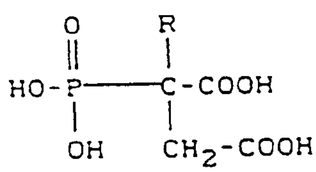

(I),

dans laquelle

R représente l'hydrogène ou un groupe alkyle en $C_1$-$C_{12}$, alcényle en $C_2$-$C_{12}$, cycloalkyle en $C_5$-$C_8$, aryle en $C_6$-$C_{12}$ ou aralkyle en $C_7$-$C_{12}$ éventuellement substitué par des goupes hydroxy, carboxy et/ou les groupes

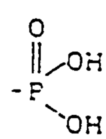

et/ou -COOR'

R' représentant l'hydrogène ou un groupe alkyle en $C_1$-$C_{12}$,

et/ou leurs sels,

la solution présentant un pH dans l'intervalle de 1 à 8.

2. Liquides selon la revendication 1, caractérisés en ce qu'ils ont un pH dans l'intervalle de 2 à 7.

3. Liquides selon les revendications 1 et 2, caractérisés en ce qu'ils contiennent un acide organique carboxylique présentant une valeur de $pK_a$ inférieure à 5.

4. Liquides selon les revendications 1 à 3, caractérisés en ce qu'ils contiennent un dérivé aminé amphotère présentant une valeur de $pK_a$ dans l'intervalle de 9,0 à 10,6 et une valeur de $pK_b$ dans l'intervalle de 11,5 à 12,5.

5. Liquides selon les revendications 1 à 4, caractérisés en ce qu'ils contiennent de l'acide éthylène-diaminetétracétique et/ou ses sels.

6. Procédé de préparation de liquides pour la préparation de la matière des dents ou des os à une restauration, caractérisé en ce que l'on prépare une solution aqueuse d'acides phosphonocarboxyliques de formule

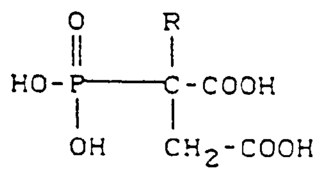

(I),

dans laquelle
R représente l'hydrogène ou un groupe alkyle en $C_1$-$C_{12}$, alcényle en $C_2$-$C_{12}$, cycloalkyle en $C_5$-$C_8$, aryle en $C_6$-$C_{12}$ ou aralkyle en $C_7$-$C_{12}$ éventuellement substitué par des groupes hydroxy, carboxy et/ou les groupes

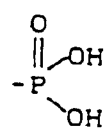

et/ou -COOR'
R' représentant l'hydrogène ou un groupe alkyle en $C_1$-$C_{12}$,
et/ou leurs sels,
en maintenant un pH dans l'intervalle de 1 à 8.

7. Utilisation des liquides selon les revendications 1 à 5 en tant que bases pour l'application de produits pour l'obturation de cavités des dents ou des os par une résine synthétique.

**Revendications pour l'Etat contractant suivant : ES**

1. Procédé de préparation de liquides prévus pour la préparation de la matière des dents ou des os à une restauration, caractérisé en ce que l'on prépare une solution aqueuse d'acides phosphonocarboxyliques de formule :

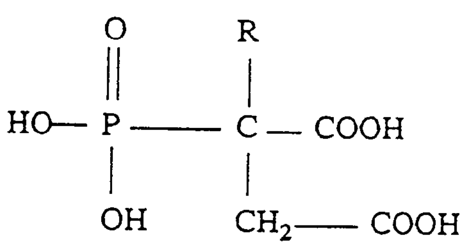

(I),

dans laquelle
R représente l'hydrogène ou un groupe alkyle en $C_1$-$C_{12}$, alcényle en $C_2$-$C_{12}$, cycloalkyle en $C_5$-$C_8$, aryle en $C_6$-$C_{12}$ ou aralkyle en $C_7$-$C_{12}$ éventuellement substitué par des groupes hydroxy, carboxy et/ou les grou-

pes

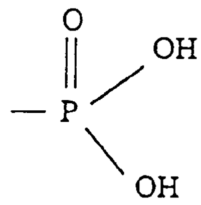

et/ou -COOR'
R' représentant l'hydrogène ou un groupe alkyle en $C_1$-$C_{12}$,
et/ou leurs sels,
en maintenant un pH dans l'intervalle de 1 à 8.

2. Procédé selon la revendication 1, caractérisé en ce que l'on maintient un pH dans l'intervalle de 2 à 7.

3. Procédé selon les revendications 1 et 2, caractérisé en ce que le liquide contient un acide organique carboxylique présentant une valeur de $pK_a$ inférieure à 5.

4. Procédé selon les revendications 1 à 3, caractérisé en ce que le liquide contient un composé aminé amphotère présentant une valeur de $pK_a$ dans l'intervalle de 9,0 à 10,6 et une valeur de $pK_b$ dans l'intervalle de 11,5 à 12,5.

5. Procédé selon les revendications 1 à 4, caractérisé en ce que le liquide contient de l'acide éthylène-diamine-tétracétique et/ou ses sels.